# EUROPEAN PATENT SPECIFICATION

(11) **EP 1 064 397 B1**
(45) Date of publication and mention of the grant of the patent: **16.01.2002**
(21) Application number: 99915566.6
(22) Date of filing: 08.03.1999
(51) Int. Cl.: C12Q 1/60, G01N 33/92, C12Q 1/00, G01N 33/53

(54) **ENZYME- AND IMMUNOASSAYS IN SURFACTANT-CONTAINING COSMETIC CLEANSERS**
ENZYM- UND IMMUNOASSAYS IN TENSID-ENTHALTENDEN KOSMETISCHEN REINIGUNGSMITTELN
PROCEDE D'IDENTIFICATION DE MATIERE ORGANIQUE

(30) Priority: 20.03.1998 EP 98302135
(43) Date of publication of application: 03.01.2001
(73) Proprietor: UNILEVER PLC, London EC4P 4BQ (GB); UNILEVER N.V., 3013 AL Rotterdam (NL)
(72) Inventor: DITTMER, Monika, D-21614 Buxtehude (DE); KRUSE, Jan, D-21614 Buxtehude (DE); MEYER, Frank, D-21614 Buxtehude (DE)
(74) Representative: Mulder, Cornelis Willem Reinier, Dr.
(86) International application number: EP9901564
(87) International publication number: WO9949078

(56) References cited:
- EP-A- 0 402 094
- WO-A-85/04423
- US-A- 4 524 133
- US-A- 5 250 418
- US-A- 5 409 664
- SCHWARZHOFF, R.; CODY, J. T. : "Effects of adulterating agents on FPIA analysis of urine for drugs of abuse" JOURNAL OF ANALYTICAL TOXICOLOGY, vol. 17, no. 1, 1993, pages 14-17, XP002109468
- S L MIKKELSEN, K O ASH: "Adulterants Causing False Negatives in Illicit Drug Testing" CLINICAL CHEMISTRY, vol. 34, no. 11, 1988, pages 2333-2336, XP002109469
- BRONNER, WILLIAM; NYMAN, PATRICIA; VON MINDEN, DAVID: "Detectability of phencyclidine and 11-nor-.DELTA.9-tetrahydrocannabinol-9-car boxylic acid in adulterated urine by radioimmunoassay and fluorescence polarization immunoassay " JOURNAL OF ANALYTICAL TOXICOLOGY, vol. 14, no. 6, 1990, pages 368-371, XP002109470

## Description

The present invention relates to the use of a biological assay for identifying organic material. In particular, the invention relates to such use for identifying presence of lipids, especially lipids that are naturally found in skin.

The surface layer of skin, the stratum corneum, contains specific lipids that are produced by the skin itself to prevent surface evaporation of water and thus prevent drying out. Cosmetic compositions, such as skin creams are conventionally used to replenish these lipids in dry skin so as to maintain a healthy skin.

Recently surfactant-containing skin cleansers have been formulated to also comprise lipids naturally found in the skin (see, for example, EP-B-0 786 903) so as to maintain the level of the skin's lipids content, even after the removal of dirt and sebum from the skin. However it appears to be difficult to test for the presence of such compounds in skin cleansers.

Although enzymatic and other biological assays are known which are able to identify a large variety of components, including lipids, in serum and other body fluids, such assays were considered to be unsuitable for testing for the presence of such compounds in surfactant containing media, because the denaturing action of surfactants would either completely destroy the enzymes involved, or so seriously reduce their catalytic activity that an effective assay would be impossible. It was also thought that other proteins such as antibodies, which can also be used to identify organic material, would also be ineffective in the presence of surfactant.

Examples of known conventional, and relatively inexpensive, tests for identifying cholesterol in serum include the liquid test kit available from Merck Inc. as the Merkoquant™ cholesterol test and that available in a strip format from Boehringer GmbH known as the AccuTrend™ test.

The reagents used in these tests are the hydrolysing and oxidising enzymes cholesterinesterase and -oxidase respectively, which together catalyse the cascade release of hydrogen peroxide from cholesterol. The presence of hydrogen peroxide can be identified by well known colour generating reactions, such as by reaction with 4-amino-antipyrine and phenols to produce coloured chinone imines.

A wide variety of colours may be generated; for example the test mentioned above from Merck provides a positive red signal, whereas that from Boehringer provides a positive blue signal.

Journal of Analytical Toxicology, Vol 17, page 14 to 17, (1993) describes the use of fluorescence polarisation immunoassays for the detection and quantification of drugs in urine samples and how the inclusion of non-ionic soap and ionic detergents in the sample interferes with the assay.

European patent application number 0,402,094 disclcses a method for measuring a substrate or an enzymatic activity in a body fluid which comprises adding an oxidase in a measuring system that includes at least one cationic and amphoteric surfactant.

International patent application WO-A-85/04423 discloses an enzyme paper for use in the indication of cholinesterase-inhibitors.

United States patent number 5,409,664 discloses a laminated bibulous diagnostic assay strip for detecting the amount of an analyte in a biological sample.

However, there is also a need for a quick and easy way of testing the presence of natural skin-lipids in surfactant-containing media.

It has now been found that sensitive biological assays can be used for accurately identifying the presence of skin-lipids and other organic compounds in surfactant-containing media, such as cosmetic cleansers. Such cosmetic cleansers, which are typically water based, include shower gals, but may also typically include other cosmetic cleansers such as liquid soaps, shower gels and foam bath compositions. Conveniently the medium contains surfactant in liquid or dissolved form.

Thus, the present invention provides a process for identifying presence of organic material in a surfactant-containing medium using a biological assay method.

In the context of this invention, a biological assay is defined as a procedure for the quantitative or qualitative identification of the presence of organic material by means of any biological or biochemical reagent capable of detecting such organic material.

Thus, according to a first aspect of the invention, the is provided a process for identifying the presence of an organic material, which is an organic material selected from lipids, carbohydrates, proteins, peptides, vitamins, organic sunscreens and mixtures thereof, in a surfactant-containing medium which contains at least 1% by weight surfactant, which biological assay comprises enzymes or immunological agents, wherein the medium contains surfactant in a liquid or dissolved form and is a cosmetic cleanser.

Conveniently, the cosmetic cleanser is a shower gel.

According to a further aspect of the invention, there is provided a process for identifying the presence of an organic material, which is an organic material selected from lipids, carbohydrates, proteins, peptides, vitamins, organic sunscreens and mixtures thereof, in a surfactant-containing medium which contains at least 1% by weight surfactant, which biological assay comprises enzymes or immunological agents, wherein the enzymes or immunological agents are immobilized on a solid support.

In a preferred embodiment, the reagents of the biological assay are enzymes, but immunological reagents such as antibodies can also be used.

In a further preferred aspect, the organic material is a lipid, and comprises one or more of sphingolipids, sterol or sterol derivatives, fatty acids or fatty acid derivatives, triglycerides, or polar lipids.

Assays can be performed in solution or by immobilising the reagents, for example on a solid support, preferably a porous strip (for instance made of paper), a porous polymeric sheet material or another support material coated with a porous layer. In a preferred use, the assays provide a quick, visual signal when organic material is identified, and such a signal can be the generation of a colour readily perceived qualitatively or may be presented quantitatively, for example photometrically. Preferred assays are based on a multiple reaction cascade format as exemplified below.

The surfactants contained in the medium in which the organic material is identified may be any surfactant (for example anionic, amphoteric, or nonionic surfactants) or combination of surfactants well known in the art (see International Cosmetic Handbook, publ.1997).

Typically the medium is a liquid and contains surfactant in liquid or dissolved form, for example as an aqueous solution of shower gel, or lather generated from such a solution. Preferably the solution or lather contains no more than 50% by wt., preferably no more than 20% by wt., more preferably no more than 10% by wt., of surfactant. Further, the solution or lather preferably contains no less than 0.1% by wt., more preferably no less than 1%, still more preferably no less than 5% of surfactant.

Although carbohydrate and protein material, peptides, vitamins, organic sunscreens or mixtures of such organic material may be identified by selecting a suitable assay which is sensitive to one or more of these materials, particularly preferred organic materials to be tested for are lipids.

Identifiable lipids may be any lipid well known in the field of cosmetic compositions (again, see the International Cosmetic Handbook mentioned above). In particular, the lipids can be natural skin-lipids, for example sphingolipids, free sterols, free fatty acids, triglycerides, or polar lipids, or mixtures of these lipids. Of the free sterols, cholesterol or their derivatives can specifically be identified.

The invention will now be illustrated by the following nonlimiting uses of enzymatic assays.

Surfactant-containing liquid cosmetic compositions (listed below) currently on the market were tested with the above mentioned Merckoquant™ and AccuTrend™ kits as follows:-

### I. The Merckoquant ™ test kit

Test solutions A (colourant I), B (colourant II) and C (enzymes) were prepared according to the procedure recommended by Merck, with a final test solution being obtained by mixing solutions A, B and 1ml of C.

The compositions to be analysed were diluted 1:1 (that is, by 50% wt. of original concentration) with de-ionized water. Subsequently, 2 ml of this diluted product and 20 ml of the above final test solution were mixed vigorously in a glass tube at room temperature. After 15 seconds a red discolouration appeared if cholesterol was present in the composition being tested; the reaction was typically complete after 10 minutes.

### II. First Test Strip

The Merckoquant™ test reagents were immobilized by dropping a few mls of the final test solution (see I. above) onto thin-layer-chromatography plates (available from Sigma-Aldrich) in the form of aluminium plates coated with silica. The so prepared test strip was immersed into an aqueous solution of the compositions (diluted by 10% wt. of original concentration) to be analyzed. When cholesterol-containing compositions were tested a reddish discolouration appeared after a few seconds (end point: 10 minutes) which remained stable over several weeks.

### III. AccuTrend™ Test Strip

A few drops of an aqueous solution of the composition (diluted by 50% wt. of original concentration) to be tested was placed on the receiving matrix in the window of the test strip. Special attention was paid to completely saturate this matrix with the diluted composition to avoid any inhomogenieity in the results of colour generated during the testing of each composition.

After 10 seconds the bluish discolouration of the test window began to appear, with the reaction being complete after 3 minutes at which point the test window was completely discoloured bluish-green.

In the following compositions, which contain natural skinlipids, a positive visual signal was generated quickly and clearly:-
Dove Cream Shower™
Rexona Sensitive™

In contrast, the following liquid products similarly tested did not give a positive visual signal:-

### Not containing natural skin-lipid

Nivea Cream Shower™
Monsavon Shower Milk™
Revlon Dry Skin Relief™ shower gel
Penaten Baby Wash™ and shower Cream™
CD Shower Balm™
Nivea Bath Care Shower Milk™
Imperial Leather Shower™ and Cream™
Garnier Nutralia 2inl™

### Not containing cholesterol

### Sanex Sport™

In a similar example, the presence or absence of glycerin was successfully tested for in shower gels using colour change assays utilizing glycerin esterase and glycerin oxidase, available commercially from Aldrich, which were immobilized on solid strips.

## Claims

1. A process for identifying the presence of an organic material, which is an organic material selected from lipids, carbohydrates, proteins, peptides, vitamins, organic sunscreens and mixtures thereof, in a surfactant-containing medium which contains at least 1% by weight surfactant, which comprises a biological assay comprising enzymes or immunological agents, and wherein the medium contains surfactant in a liquid or dissolved form, and is a cosmetic cleanser.

2. A process according to claim 1, wherein the lipid is cholesterol or a cholesterol derivative.

3. A process according to claim 1 or claim 2, wherein the assay utilizes esterase and/or oxidase enzymes.

4. A process according to claim 3, wherein the enzymes are cholesterin-esterase, cholesterin oxidase, glycerin-esterase or glycerin oxidase.

5. A process according to any preceding claim, wherein the enzymes or immunological agents are immobilized on a solid support.

6. A process according to any preceding claim, wherein the enzymes or immunological agents provide a quick, visual signal when the organic material is identified.

7. A process according to claim 6, wherein the signal provides a quantitative measure of the amount of organic material tested for.

8. A process according to any preceding claim, wherein the medium contains anionic, amphoteric, or nonionic surfactant, or mixtures of such surfactants.

9. A process according to any preceding claim, wherein the cosmetic cleanser is a shower gel.

10. A process according to claim 9, wherein a lather from the dissolved form is tested.

11. A process according to any preceding claim, wherein the medium contains no more than 50% by wt. of surfactant.

12. A process according to claim 11, wherein the medium contains no more than 20% by wt. surfactant.

13. A process according to claim 12, wherein the medium contains no more than 10% by wt. surfactant.

14. A process according to any preceding claim, wherein the medium contains at least 5% by wt. surfactant.

15. A process according to any of claims 4-14, wherein the solid support is a porous strip.

16. A process according to claim 15, wherein the porous strip is a paper strip.

## Patentansprüche

1. Verfahren zur Identifizierung des Vorhandenseins eines organischen Materials, ausgewählt aus Lipiden, Kohlenhydraten, Proteinen, Peptiden, Vitaminen, organischen Lichtschutzmitteln und Gemischen davon, in einem Tensid-haltigen Medium, das wenigstens 1 Gewichtsprozent Tensid enthält, das einen biologischen Assay umfaßt, der Enzyme oder immunologische Mittel umfaßt und wobei das Medium das Tensid in einer flüssigen oder gelösten Form enthält und ein kosmetisches Reinigungsmittel ist.

2. Verfahren nach Anspruch 1, wobei das Lipid Cholesterin oder ein Cholesterinderivat ist.

3. Verfahren nach Anspruch 1 oder Anspruch 2, wobei das Assay Esterase und/oder Oxidaseenzyme verwendet.

4. Verfahren nach Anspruch 3, wobei die Enzyme Cholesterinesterase, Colesterinoxidase, Glycerinesterase oder Glycerinoxidase sind.

5. Verfahren nach einem der vorstehenden Ansprüche, wobei die Enzyme oder immunologischen Mittel auf einem festen Träger immobilisiert sind.

6. Verfahren nach einem der vorstehenden Ansprüche, wobei die Enzyme oder immmunologischen Mittel ein schnelles, visuelles Signal liefern, wenn das organische Material identifiziert ist.

7. Verfahren nach Anspruch 6, wobei das Signal eine quantitative Bestimmung der Menge des getesteten organischen Materials liefert.

8. Verfahren nach einem der vorstehenden Ansprüche, wobei das Medium anionische, amphotere oder nichtionische Tenside oder Gemische solcher Tenside enthält.

9. Verfahren nach einem der vorstehenden Ansprüche, wobei das kosmetische Reinigungsmittel ein Duschgel ist.

10. Verfahren nach Anspruch 9, wobei ein Schaum der gelösten Form getestet wird.

11. Verfahren nach einem der vorstehenden Ansprüche, wobei das Medium nicht mehr als 50 Gewichtsprozent Tensid enthält.

12. Verfahren nach Anspruch 11, wobei das Medium nicht mehr als 20 Gewichtsprozent Tensid enthält.

13. Verfahren nach Anspruch 12, wobei das Medium nicht mehr als 10 Gewichtsprozent Tensid enthält.

14. Verfahren nach einem der vorstehenden Ansprüche, wobei das Medium wenigstens 5 Gewichtsprozent Tensid enthält.

15. Verfahren nach einem der Ansprüche 4 - 14, wobei der feste Träger ein poröser Streifen ist.

16. Verfahren nach Anspruch 15, wobei der poröse Streifen ein Papierstereifen ist

## Revendications

1. Procédé d'identification de la présence d'une matière organique, qui est une matière organique sélectionnée à partir des lipides, des hydrates de carbone, des protéines, des peptides, des vitamines, des écrans solaires organiques et des mélanges de ceux-ci, dans un milieu contenant du tensioactif qui contient au moins 1 % en poids de tensioactif, qui comprend un test biologique comprenant des enzymes ou des agents immunologiques et dans lequel le milieu contient le tensioactif sous une forme liquide ou dissoute et est un nettoyant cosmétique.

2. Procédé selon la revendication 1, dans lequel le lipide est du cholestérol ou un dérivé de cholestérol.

3. Procédé selon la revendication 1 ou la revendication 2, dans lequel le test utilise des enzymes d'estérase et/ou d'oxydase.

4. Procédé selon la revendication 3, dans lequel les enzymes sont de la cholestérine-estérase, de la cholestérine-oxydase, de la glycérine-estérase ou de la glycérine-oxydase.

5. Procédé selon l'une quelconque des revendications précédentes, dans lequel les enzymes ou les agents immunologiques sont immobilisés sur un matériau solide formant support.

6. Procédé selon l'une quelconque des revendications précédentes, dans lequel les enzymes ou les agents immunologiques fournissent un signal visuel rapide lorsque le matériau organique est identifié.

7. Procédé selon la revendication 6, dans lequel le signal fournit une mesure quantitative de la quantité de matière organique testée.

8. Procédé selon l'une quelconque des revendications précédentes, dans lequel le milieu contient des tensioactifs anioniques, amphotères ou non ioniques, ou des mélanges de tels tensioactifs.

9. Procédé selon l'une quelconque des revendications précédentes, dans lequel le nettoyant cosmétique est un gel douche.

10. Procédé selon la revendication 9, dans lequel on teste une mousse provenant de la forme dissoute.

11. Procédé selon l'une quelconque des revendications précédentes, dans lequel le milieu ne contient pas plus de 50 % en poids de tensioactif.

12. Procédé selon la revendication 11, dans lequel le milieu testé ne contient pas plus de 20 % en poids de tensioactif.

13. Procédé selon la revendication 12, dans lequel le milieu testé ne contient pas plus de 10 % en poids de tensioactif.

14. Procédé selon la revendication 13, dans lequel le milieu testé ne contient pas plus de 5 % en poids de tensioactif.

15. Procédé selon l'une des revendications 4 à 14, dans lequel le matériau solide formant support est une bandelette poreuse.

16. Procédé selon la revendication 16, dans lequel la bandelette poreuse est une bandelette de papier.
